# EUROPEAN PATENT SPECIFICATION

(11) **EP 2 710 120 B1**
(45) Date of publication and mention of the grant of the patent: **29.03.2017**
(21) Application number: 12785487.5
(22) Date of filing: 17.05.2012
(51) Int. Cl.: C12N 5/071, C12M 1/34

(54) **STABLE ELECTRICALLY ACTIVE NEURONS FROM ADULT TISSUE**
STABILE ELEKTRISCH AKTIVE NEURONEN AUS ADULTEM GEWEBE
NEURONES ÉLECTRIQUEMENT ACTIFS STABLES PROVENANT DE TISSUS ADULTES

(30) Priority: 17.05.2011 US 201161487251 P
(43) Date of publication of application: 26.03.2014
(73) Proprietor: University of Central Florida Research Foundation, Inc., Orlando, FL 32826 (US)
(72) Inventor: HICKMAN, James, Orlando, FL 32817 (US); EDWARDS, Darin, Orlando, FL 32817 (US)
(74) Representative: Wibbelmann, Jobst
(86) International application number: PCT/US2012/038358
(87) International publication number: WO 2012/158923

(56) References cited:
- WO-A1-2007/118984
- US-A1- 2007 015 138
- US-A1- 2008 124 789
- F. X. SORIANO: "Preconditioning Doses of NMDA Promote Neuroprotection by Enhancing Neuronal Excitability", JOURNAL OF NEUROSCIENCE, vol. 26, no. 17, 26 April 2006 (2006-04-26), pages 4509-4518, XP055149808, ISSN: 0270-6474, DOI: 10.1523/JNEUROSCI.0455-06.2006
- Lippincott Williams ET AL: "Effects of Topiramate on Sustained Repetitive Firing and Spontaneous Recurrent Seizure Discharges in Cultured Hippocampal Neurons", Epilepsia, 1 January 2000 (2000-01-01), pages 4-44, XP055154810, Retrieved from the Internet: URL:http://onlinelibrary.wiley.com/store/1 0.1111/j.1528-1157.2000.tb02170.x/asset/j. 1528-1157.2000.tb02170.x.pdf?v=1&t=i2x4e8v p&s=d679fc7d2fa69a82388fefe3c40781a74102c9 e5 [retrieved on 2014-11-25]
- UK NAMGUNG ET AL: "Activation of cyclin-dependent kinase 5 is involved in axonal regeneration", MOLECULAR AND CELLULAR NEUROSCIENCE, vol. 25, no. 3, 1 March 2004 (2004-03-01), pages 422-432, XP055154813, ISSN: 1044-7431, DOI: 10.1016/j.mcn.2003.11.005
- FRANK GILLARDON ET AL: "Investigating the neuroprotective mechanism of action of a CDK5 inhibitor by phosphoproteome analysis", JOURNAL OF CELLULAR BIOCHEMISTRY, vol. 95, no. 4, 1 January 2005 (2005-01-01), pages 817-826, XP055154816, ISSN: 0730-2312, DOI: 10.1002/jcb.20463
- J WEISHAUPT: "Inhibition of CDK5 is protective in necrotic and apoptotic paradigms of neuronal cell death and prevents mitochondrial dysfunction", MOLECULAR AND CELLULAR NEUROSCIENCE, vol. 24, no. 2, 1 October 2003 (2003-10-01), pages 489-502, XP055154819, ISSN: 1044-7431, DOI: 10.1016/S1044-7431(03)00221-5
- YAN Z ET AL: "Roscovitine: A novel regulator of P/Q-type calcium channels and transmitter release in central neurons", THE JOURNAL OF PHYSIOLOGY, WILEY-BLACKWELL, US, vol. 540, no. 3, 1 May 2002 (2002-05-01), pages 761-770, XP002485997, ISSN: 0022-3751, DOI: 10.1113/JPHYSIOL.2001.013376
- FRANK GILLARDON ET AL: "Phosphoproteome and transcriptome analysis of the neuronal response to a CDK5 inhibitor", PROTEOMICS, vol. 5, no. 5, 1 April 2005 (2005-04-01), pages 1299-1307, XP055154818, ISSN: 1615-9853, DOI: 10.1002/pmic.200400992
- James A Bibb ET AL: "PROTEIN PHOSPHATASE INHIBITOR-1 AND CDK5: OF MOLECULES AND MEMORY APPROVED BY SUPERVISORY COMMITTEE", Ph.D. Thesis University of Texas Southwestern Medical center Dallas, 1 April 2007 (2007-04-01), pages 1-202, XP055147644, Retrieved from the Internet: URL:http://repositories.tdl.org/utswmed-ir /bitstream/handle/2152.5/409/nguyenbaochan .pdf?sequence=3 [retrieved on 2014-10-20]
- BERMEL C ET AL: "Combined inhibition of Cdk5 and ROCK additively increase cell survival, but not the regenerative response in regenerating retinal ganglion cells", MOLECULAR AND CELLULAR NEUROSCIENCES, SAN DIEGO, US, vol. 42, no. 4, 1 November 2009 (2009-11-01), pages 427-437, XP026751406, ISSN: 1044-7431, DOI: 10.1016/J.MCN.2009.09.005 [retrieved on 2009-09-25]
- ZELDA H. CHEUNG ET AL: "Cdk5: mediator of neuronal death and survival", NEUROSCIENCE LETTERS, vol. 361, no. 1-3, 1 May 2004 (2004-05-01) , pages 47-51, XP055154822, ISSN: 0304-3940, DOI: 10.1016/j.neulet.2003.12.117
- MONACO III ET AL.: 'Roscovitine Triggers Excitotoxicity in Cultured Granule Neurons by Enhancing Glutamate Release.' MOLECULAR PHARMOCOL. vol. 68, no. 5, November 2005, pages 1331 - 1342, XP055136764
- VARGHESE ET AL.: 'Regeneration and characterization of adult mouse hippocampal neurons in a defined in vitro system.' JOURNAL OF NEUROSCIENCE METHODS. vol. 177, no. 1, 15 February 2009, pages 51 - 59, XP025841264

## Description

### ACKNOWLEDGEMENTS

This invention was made with government support under Grant R1 EB005459 awarded by NIH. The government has certain rights in the invention.

### CROSS REFERENCE TO RELATED APPLICATIONS

This application claims the benefit of U.S. Provisional Patent Application Serial Number 61/487,251, filed on May 17,2011

### FIELD OF THE INVENTION

This invention relates generally to the field of *in vitro* cell culture systems and microelectrode arrays. Specifically, the invention relates to neuronal/microelectrode array hybrid systems.

### BACKGROUND OF THE INVENTION

Neurodegenerative conditions as well as traumatic brain injury (TBI) are characterized by widespread loss of functional, active neurons. Investigations into treatments and therapies for these conditions are reliant upon the existence of accurate systems mimicking the conditions found in the human brain. Widely used systems include rat and mouse disease models mimicking pathological symptoms of the diseases, embryonic neuron cell culture systems including dissociated cell culture and brain slices, and later stage testing on human subjects. Some work has been performed on the refinement of *in vitro* dissociated neuronal systems to use adult brain tissue rather than embryonic brain tissue. Problems exist with these current *in vitro* culture systems. Difficulty in limiting damage during the dissociation of adult brain tissue, mitigation of cellular trauma from the release of myelin inhibitory proteins, problems promoting both regeneration and long-term survival of adult brain-derived neurons, and proliferation of previously quiescent terminally differentiated neurons are among the most common problems encountered in creating such an *in vitro* system.

Adult central nervous system neurons have long been described as post mitotic, with neurons arrested in a G0 phase of the cell cycle. However, this cell cycle arrest in neurons is not permanent, but rather, mature differentiated neurons must constantly regulate themselves to keep from progressing through the cell cycle. *In vitro,* post mitotic neurons revert to a proliferative state due to the action of the essential factor basic fibroblast growth factor (bFGF) .

The factor bFGF also promotes GABA-negative neurons to survive by influencing both glucosyceramide synthesis and the VDCCs. It also up-regulates cdk5 expression, triggering neuronal re-entry into the cell cycle. Cdk5, while generally expressed in neurons in the cdk5/p35 complex, is normally expressed by neurons at levels that exert an influence on neurite outgrowth and migration but not proliferation. Cyclin-dependent kinase inhibitors (CKIs), which include the competitive cdk5/p35 inhibitor roscovitine, normally play an important role in regulatory decisions controlling progression through the cell cycle.

Electrophysiological studies into learning and memory formation in the presence of neurotoxic compounds and under neurodegenerative conditions most commonly rely upon patch-clamp electrophysiology. While this method provides detailed information, it is very labor-intensive, complicated, and has limited long-term capability in relation to non-invasive techniques for measuring electrical activity such as microelectrode arrays (MEAs). MEAs are innovative tools used to perform electrophysiological experiments for the study of both neuronal activity and connectivity in populations of neurons from dissociated cultures. Where patch-clamp electrophysiology can only measure the electrical activity of cells over the short-term (< few hours), MEA systems can measure the same population of neurons and the chronic effect of toxic compounds, drugs, etc on those neurons for extended periods of time (days - months). Uses for these systems include studies into the mechanisms of learning and memory formation and investigations into drug discovery, neurodegenerative diseases, and biosensor applications.

A common limitation of neuronal MEA systems as well as neuroscience research in general has been the reliance upon neurons derived from embryonic tissue. While these are differentiated neurons, they are developmentally immature, with transcriptional profiling indicating two-thirds of genes are only expressed postnatal and >95% of the expressed genes show highly significant changes during postnatal development. In fact, the electrophysiological properties of embryonic or neonatal neurons yield promising *in vitro* epilepsy models. When evaluating the machinery responsible for synaptic transmission, AMPA channel expression is limited at birth, only increasing post-natally. The gene expression for the NMDA channel subunits NR2A and NR2B was not detected until near birth at E21-22, with expression not peaking until P20 14. *In vitro,* NR2A/B channels are detected at only very low levels until after 2 weeks in embryonically derived neurons. Gene expression for the axonal sodium transporter subunit 1 begins around P15, then increasing until P30. *In vitro,* expression patterns for all genes, specifically axonal and synaptic channels responsible for signal transmission, is similar to that seen *in vivo,* with gene expression showing significant changes over the course of weeks. The usefulness of these developmentally immature neurons for studies of neuronal electrical activity and synaptic transmission has been restricted by the limited expression or lack of expression of certain channels responsible for synaptic transmission in the adult brain. In addition, using these developmentally immature neurons in studies of neurodegenerative diseases or drug discovery can yield results that are difficult to correlate with the function or action of mature neurons in adult brain tissue.

Soriano et al. disclose in "Preconditioning doses of NMDA promote neuroprotection by enhancing neuronal excitability", J. Neurosci, 2006, 26(17), 4509-18 that exposure to low preconditioning doses of NMDA results in preferential activation of synaptic NMDA receptors because of a dramatic increase in action potential firing.

DeLorenzo et al. disclose in "Effects of Topiramate on Sustained Repetitive Firing and Spontaneous Recurrent Seizure Discharges in Cultured Hippocampal Neurons", Epilepsia, 2000, 41, 40-44 that topimarate exerts modulatory effects on voltage-dependent Na⁺ and/or Ca²⁺ conductances responsible for the generation and propagation of action potentials and may inhibit synaptic conductances responsible for transmission of epileptiform discharges.

Namgung et al. disclose in "Activation of cyclin-dependent kinase 5 is involved in axonal regeneration", Mol. Cell Neurosci. 2004, 2, 422-32 a high expression of CDK-5 and p35 in regenerating nerves.

Gillardon et al. report in "Investigating the neuroprotective mechanism of action of a CDK5 inhibitor by phosphoproteome analysis", J. Cell Biochem., 2005, 95(4), 817-26 that inhibition of CDK5 activates stress responsive proteins that may protect neurons against subsequent injurious stimuli.

Weishaupt et al. disclose in "Inhibition of CDK5 is protective in necrotic and apoptotic paradigms of neuronal cell death and prevents mitochondrial dysfunction", Mol. Cell. Neurosci., 2003, 24, 489-502 that CDK5 activates neuronal cell death pathways upstream of mitochondrial dysfunction, and inhibition of CDK5 may promote functional long-term rescue of injured neurons.

What is needed in the art is a dissociated neuronal cell culture system that can be used with microelectrode arrays, in which the neuronal cells have the functional characteristics of adult neurons for studying the function of neurons, neuronal interactions, aging, neurodegenerative diseases, drug studies, neuroprosthetic devices, neurocomputing, biorobotics, and neuroregeneration, especially in traumatic brain injury.

### SUMMARY

In accordance with the purpose(s) of this invention, as embodied and broadly described herein, the present invention comprises methods of producing a population of electrically active neurons, comprising contacting adult neural cells with a cdk5 inhibitor to control cell division of the adult neural cells and contacting the adult neural cells with a neurotransmitter, thereby producing a population of electrically active neurons.

In another aspect, the present invention comprises methods of utilizing adult neurons for testing purposes, comprising contacting at least one electrically active adult neuron, or a membrane portion thereof, with a test compound, the at least one electrically active adult neuron having been cultured under conditions where cell division is controlled, and observing for an effect of the test compound on the at least one electrically active adult neuron, or membrane portion thereof.

In another aspect, disclosed are microelectrode array systems comprising a microelectrode array having a surface and at least one electrically active adult neuron disposed on a surface thereof, the at least one electrically active adult neuron being derived from adult nervous tissue.

In another aspect, disclosed are populations of electrically active neurons, wherein the neurons were produced by contacting adult neural cells with a cdk5 inhibitor to control cell division of the adult neural cells and contacting the adult neural cells with a neurotransmitter under culture conditions to promote electrical function.

In another aspect, disclosed are methods of identifying test compounds having a possible effect on an electrically active adult neuron, comprising contacting a microelectrode array system with a test compound candidate, the microelectrode array system comprising a microelectrode array having a surface and at least one electrically active adult neuron disposed on a surface thereof, the at least one electrically active adult neuron cell being derived from adult nervous tissue, and observing for an effect of the test compound on the microelectrode array system, at least one electrically active adult neuron cell, or membrane portion thereof.

In yet another aspect, disclosed are microscale fluid handling systems, comprising a microfluidic device; and at least one three dimensional multi-cellular surrogate tissue assembly comprising a population of electrically active neurons, wherein the device is used for initiating, culturing, manipulating, and assaying each tissue assembly of the system.

Additional advantages of the invention will be set forth in part in the description which follows, and in part will be obvious from the description, or may be learned by practice of the invention. The advantages of the invention will be realized and attained by means of the elements and combinations particularly pointed out in the appended claims.

### BRIEF DESCRIPTION OF THE DRAWINGS

The accompanying drawings, which are incorporated in and constitute a part of this specification, illustrate (one) several embodiment(s) of the invention and together with the description, serve to explain the principles of the invention.

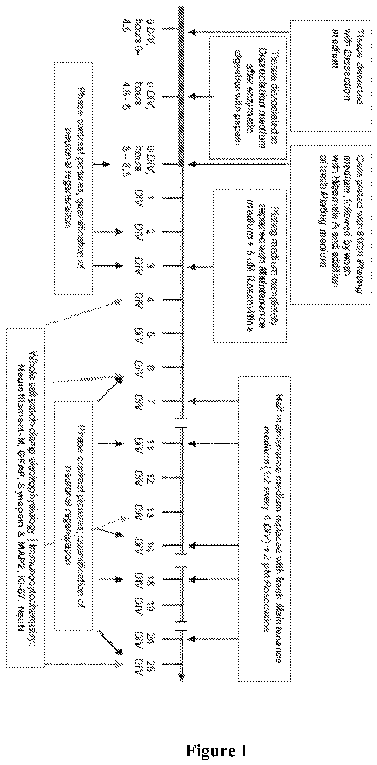

Figure 1 shows culture methodology for processing and dissociation of adult rat hippocampal tissue to create and maintain a dissociated neuronal cell culture. The time scale of the cell culture shows timed application of growth factors and quantification of various neuronal parameters. The cells were examined immunocytochemically after 6,14, and 25 days *in vitro* (Neurofilament-M, Ki-67, NeuN, MAP2, Synapsin, and GFAP). Electrical parameters of cells were examined after 6, 13, and 25 days *in vitro.* Cell division examined with and without introduction of Roscovitine between 1-80 days *in vitro.*

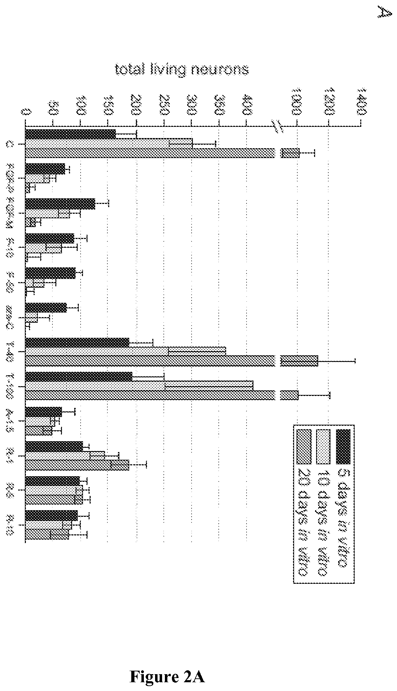

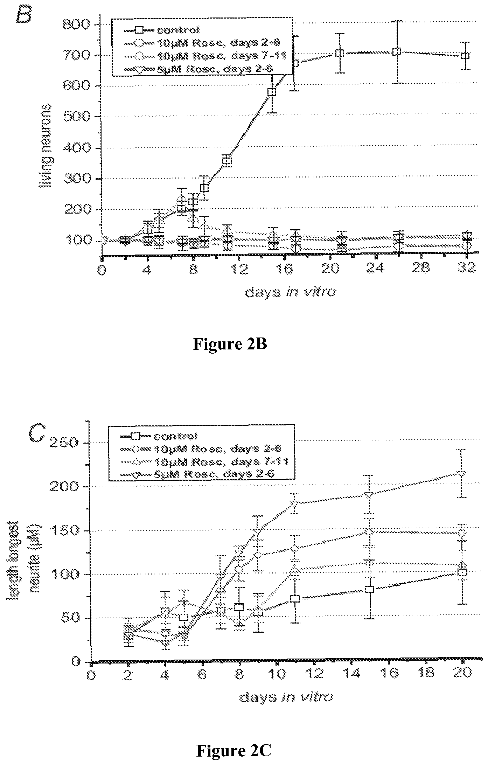

Figures 2A, 2B, and 2C show roscovitine (Rose) prevents mature adult hippocampal neurons from returning to the cell cycle in the presence of bFGF and dividing *in vitro.* A. Neuronal population after growth factor removal or addition of mitotic factors. All day 0 cell densities normalized to 100 to allow for comparison. C - control, standard medium and factors, FGF-P - bFGF removed from both the plating and maintenance medium, FGF-M - bFGF removed from the maintenance medium, F-10 - 10 µM FudR on 2 DIV, F-50 - 50 µM FudR on 2 DIV, ara-C - ara-C on 2 DIV, T-40-40 µM Trolox on 2 DIV, T-100-100 µM Trolox on 2 DIV, A-1.5 - 1.5 µM Aph on 2 DIV, R-1-1 µM Rose on 2 DIV, R-5 - 5 µM Rose on 2 DIV, R-10 - 10 µM Rose on 2 DIV. B, C: Effect on neuronal cell division and neurite length from the addition of different concentrations of Rosc *in vitro.* All day 0 cell densities normalized to 100 to allow for comparison. Control - neurons were not treated. Group 1-neurons treated with 10µM Rosc days 2-6, 2 µM Rosc after day 6. Group 2, neurons treated with 10 µM Rosc days 7-11,2µM Rosc after day 11. Group 3, neurons treated with 5 µM Rose days 2-6, 2 µM Rosc after day 6. Adult neurons not treated with Rosc divide *in vitro* until confluent, while the population of neurons after treatment with 5 µM Rosc was stable *in vitro.* Neurons express MAP2, NeuN, do not express Ki-67 *in vitro* in the presence of 5 µM Rosc.

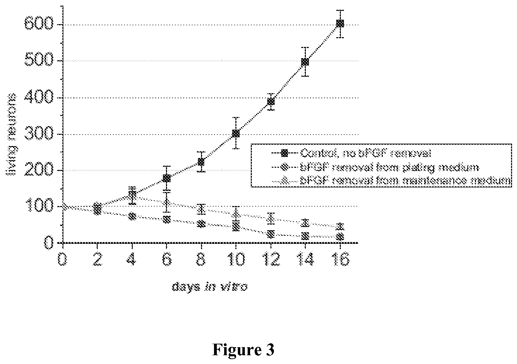

Figure 3 shows effect from bFGF removal on neuronal survival and proliferation. bFGF in culture medium caused neurons to divide *in vitro,* while removal of bFGF caused neuronal loss through apoptosis and necrosis. All day 0 cell densities normalized to 100 to allow for comparison. Removal of bFGF from the culture media prevented neuronal division but triggered apoptosis. 6, 7, and 8 days *in vitro* in neuronal populations grown in media without bFGF.

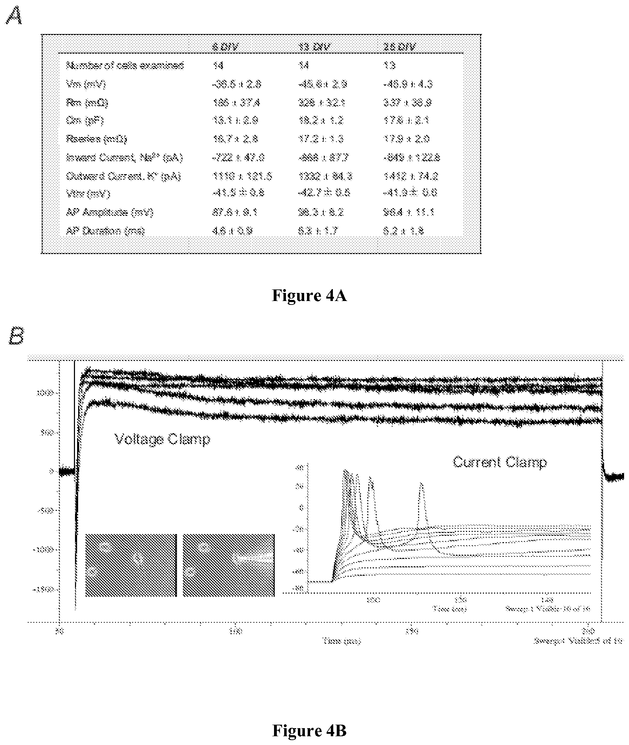

Figures 4A and 4B show A, electrical properties of adult rat hippocampal neurons after 6, 13, or 25 days *in vitro,* examined through whole-cell patch-clamp electrophysiology. DIV=culture days *in vitro,* Vm=resting membrane potential, Rm=membrane resistance, Cm=membrane capacitance, Rseries=series resistance, Vthr=action potential threshold voltage, AP=action potential. Data is presented as mean +/- S.E.M. B, Representative trace for voltage and current clamp of an adult neuron firing a single action potential as well as moving current into and out of the cell through voltage-gated ion channels. These traces originated from a adult rat hippocampal neurons after 13 DIV.

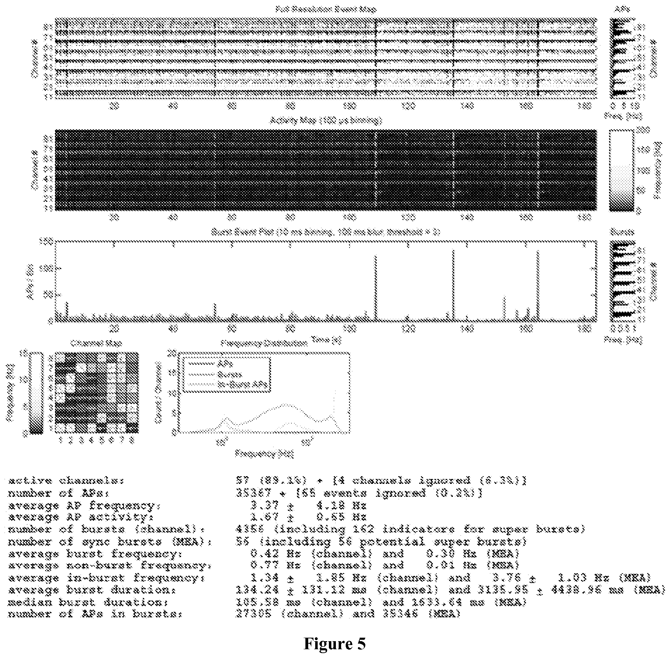

Figure 5 shows MEA data processing: MEA data processing: The spontaneous activity of embryonic and adult neurons was recorded for 3 minutes each day over the study period. Each 3 minute dataset was processed in a 3-step method. This method allowed inactive or noisy channels to be excluded. After processing, the following parameters were extrapolated: "active channels" - a number from 0 to 64. Channels with less than 7.5 APs per minute were treated as inactive. "AP Frequency" - number of total measured APs divided by recording time. The mean and stdev of that number is independent from active channels. "AP Activity" - is 1 divided by the time between two subsequent APs, or frequency 1/s. "Burst" - where more than 1 AP shows up with 1 ms. "average burst frequency" - 1 divided by the time-difference between two subsequent bursts. "In-burst frequency" - amount of APs within a burst divided by the duration of that burst. "Non-burst frequency" - like the activity, but considers on APs that are not associated with bursts. "Burst duration" - time-interval from the first AP in a burst to the last AP in a burst.

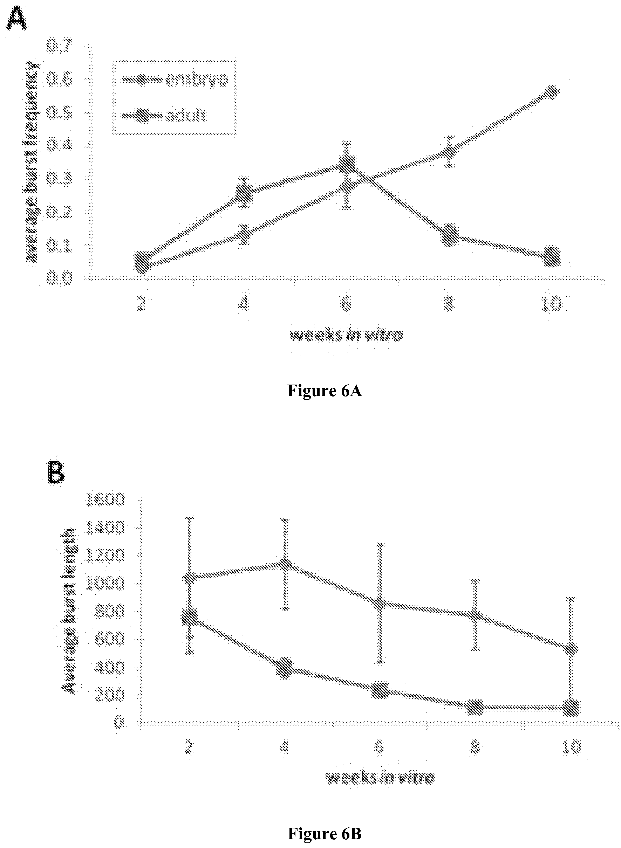

Figures 6A-6D show basic firing patterns of embryonic and adult hippocampal neurons on MEAs over time. The spontaneous activity of embryonic and adult neurons was recorded for 3 minutes each day over the study period. The activity data from each day were processed to filter out inactive channels. (A) Average burst frequency. (B) Average burst length. (C) Average firing frequency within a burst. (D) Average firing frequency outside of bursts.

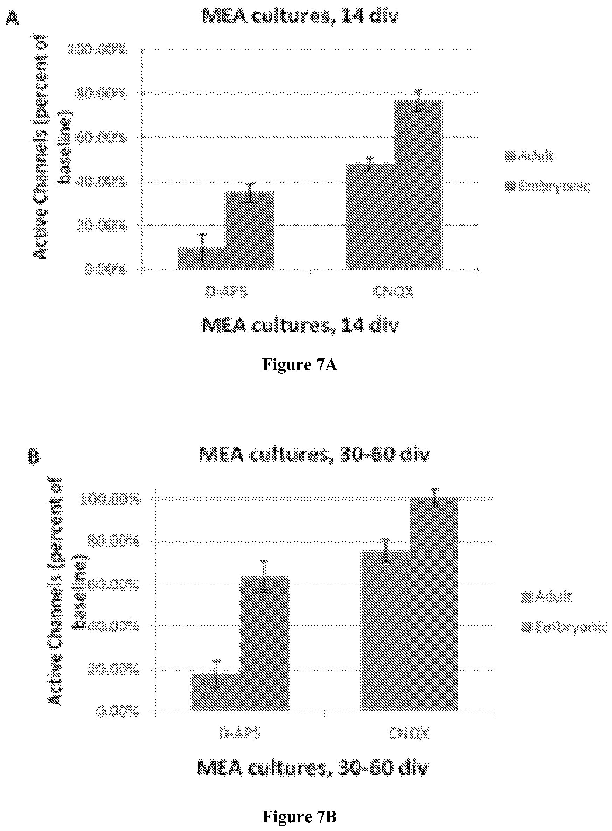

Figures 7A-7D show comparison of the impact on adult or embryonic spontaneous activity from addition of synaptic antagonists. Active channels (A,B) or AP frequency (C,D) were evaluated in adult or embryonic hippocampal neuron MEA systems on either 14 or 30-60 div in the presence of D-AP5 (25 µM) or CNQX (25 µM) in culture medium. Adult neurons showed significantly decreased active channels and AP frequency due to D-AP5 in both early 14 div cultures as well as older 30-60 div cultures. This drop in activity was significantly different from embryonic 14 div, where fewer active channels were lost and activity increased in the remaining channels. The AMPA-channel antagonist CNQX also caused a decrease in spontaneous activity. The drop in activity between adult and embryonic cultures was, however, only reflected in the loss of more active channels in the adult system. AP frequency declines were consistent between the two culture systems.

### DETAILED DESCRIPTION

The present invention may be understood more readily by reference to the following detailed description of preferred embodiments of the invention and the Examples included therein and to the Figures and their previous and following description.

The present invention comprises methods comprising exposing adult neural cells to a cdk5 inhibitor to control cell division of the neural cells. The method further involves encouraging development of electrical function of the neural cells by subjecting the neural cells to a neurotransmitter. Adult neural cells may be electrically functioning prior to exposure to a neurotransmitter.

The present invention comprises compositions of and methods for utilizing electrically active adult neural cells for testing and/or screening purposes. In an aspect, a method can involve exposing at least one electrically active adult neuron, or a membrane portion thereof, to a test compound and observing for an effect of the test compound on the at least one electrically active adult neuron, or membrane portion thereof. A person of ordinary skill in the art can observe the electrophysiology of the at least one electrically active adult neuron, or membrane portion thereof, before and after exposure of the at least one electrically active adult neuron cell, or membrane portion thereof, to a test compound. For example, a person of ordinary skill in the art can observe up-modulation or down-modulation of ion flow across the cell membrane of the at least one electrically active adult neuron cell, or membrane portion thereof, indicating that the test compound modulates electrical activity. Other non-limiting examples of effects that can be observed may include changes in frequency and/or amplitude of action potentials, longevity, robustness (health), or protection against the presence of a toxin or other harmful or disease causing agents. Source material for adult neurons may be normal neural tissue, neural tissue from a known disease state, or neural tissue from a suspected or unknown disease state.

The present invention comprises dissociated neuronal cell culture systems derived from neural tissue to produce a stable population of electrically active neurons. Through the action of regeneration-promoting growth factors, for example, basic Fibroblast Growth Factor (bFGF) and the dose-dependent application of anti-mitotic factors, cdk5 mediated cell cycle progression can be activated or deactivated to promote or control the division of adult, terminally differentiated neurons. The application of cell cycle control compositions to an improved serum-free culture system allows for either the expansion of primary adult neuronal cells under controlled conditions or for the development of a stable population of primary neurons that both morphologically and functionally regenerate without expansion. These neurons may be applied to culture surfaces coated with the chemical substrate N-1 [3-(trimethoxysilyl) propyl]-diethylenetriamine (DETA), a surface previously shown to be superior in the promotion of attachment, regeneration and long-term survival of neurons *in vitro* (D. A. Stenger et al., 1993; J. J. Hickman et al., 1994; M. Ravenscroft et al., 1998; M. Das et al., 2005; D. Edwards et al., 2010; A. E. Schaffner et al., 1995). The Examples show an exemplary system comprising a neuronal cell culture system derived from the hippocampus of adult rats.

As used herein, the term "adult" in the context of describing cells or tissue relates to cells that are terminally differentiated, or tissue containing such cells. The term "adult" in the context of describing a subject (human or nonhuman animal) typically means an animal that has reached reproductive capacity. While adult cells may be obtained from an adult subject, cells and/or tissue may be obtained from a non-adult subject and still be considered adult cells and/or tissue so long as the non-adult subject is post-natal and the adult cells and/or tissue are terminally differentiated.

As used herein, a "subject" is an individual and includes, but is not limited to, a mammal (*e.g.,* a human, horse, pig, rabbit, dog, sheep, goat, non-human primate, cow, cat, guinea pig, rat, mouse, or other rodents), a fish, a bird, a reptile or an amphibian. The term does not denote a particular age or sex. Thus, adult and newborn subjects, as well as fetuses, whether male or female, are intended to be included. A "patient" is a subject afflicted with a disease or disorder. The term "patient" includes human and veterinary subjects.

As used herein, a "neuron" is a "neural cell." The terms can be used interchangeably.

The present invention comprises methods comprising obtaining adult neural cells (neurons) from a tissue sample and exposing the obtained adult neural cells to a growth factor under suitable conditions to induce cell division of the neural cells. Other neural cells, such as glial cells, support cells, Schwann cells or other cells found in the brain or spinal cord, or cells known to those skilled in the art as derived from neural tissue may be included with neural cells in sampling or in culturing. Induction of cell division typically occurs prior to a step of controlling cell division by exposure to a cdk5 inhibitor. The cells may be maintained in the presence of both a growth factor and a cdk5 inhibitor.

Adult neural cells can be obtained by the dissection and enzymatic digestion of, for example, an adult neural tissue in the presence of a dissecting medium. After cells have been obtained from the tissue sample, they can be separated from each other by mechanical dissociation of the tissue sample. The individual adult neural cells can then be plated (attached) to a substrate in the presence of a plating medium for a desired period of time, for example three days. The cells on the substrate can be maintained from, for example day 3 of the culture, through the end of the culture. Exemplary methods of culturing cells are provided in the Examples herein, and those skilled in the art can determine periods of time for growing and attaching cells, and for exposure to particular media.

The present invention comprises methods for producing a population of electrically active (functional) neurons, comprising a) contacting adult neural cells with a growth factor, b) contacting the neural cells of step a) with a cdk5 inhibitor, and then c) contacting the neural cells of step b) with a neurotransmitter, wherein the adult neural cells develop into a population of electrically active neurons. Non-limiting examples of neurotransmitters include acetylcholine, dopamine, serotonin (5-HT), norepinephrine, epinephrine, GABA, prolactin, nitric oxide, histamine (H1, H2, H3, H4), vasopressin, oxytocin, endocannabinoids, endogenous opioids (Mu-1, Mu-2, kappa, delta, ORL, sigma receptors), glycine, and GHB.

Adult neural cells are cells taken from a sample of adult neural tissue. In an aspect, adult neural tissue is adult tissue from the central nervous system of a subject. Thus, adult neural tissue can be tissue from the brain or spinal cord of a subject. In an aspect, adult neural tissue can be hippocampal tissue from the brain of a subject. The peripheral nervous system may be a source for adult neural tissue.

Contacting adult neural cells with a growth factor under suitable conditions for a period of time induces the cells to divide in order to achieve confluence on a substrate. In some aspects, the cells may reach confluence without a growth factor present. A time for contact by the growth factor may be in a range of from 1 hour to about 2 days, to about 3 days, to about 5 days, to about 10 days or longer, and any time period thereinbetween. A non-limiting example of a growth factor is basic fibroblast growth factor (bFGF). In an aspect, a substrate, for example a glass cover slip, can be treated with N-1 [3-(trimethoxysilyl) propyl]-diethylenetriamine (DETA), resulting in a cell adhesive surface. Applying dissociated adult neural cells to a DETA-treated glass cover slip in a suitable plating medium allows neurons to attach with minimal attachment of cellular debris.

After adult electrically active neural cells are attached to a substrate, the cells can be maintained by being in contact with a maintenance medium, which can comprise a growth factor and a cdk5 inhibitor. Non-limiting examples of an inhibitor of cdk5 include butyrolactone. indolinone A and roscovitine.

Figure 1 shows an exemplary cell culture methodology for processing and dissociating adult rat hippocampal tissue to create and maintain a dissociated neuronal cell culture. Table 1 lists the compositions of the dissecting medium, the dissociation medium, the plating medium, and the maintenance medium that are needed to produce a population of electrically active adult neural cells.

A population of adult electrically active neurons can be created by dissecting adult neural cells. Shown in the Examples is a population of adult electrically active neurons from rat hippocampal tissue in the presence of a dissecting medium. See Table 1 for the composition of a dissecting medium. After a period of time, for example four to five hours, the adult neural cells can then be dissociated from each other in the presence of a dissociation medium. Approximately one to two hours later, the dissociated cells can then be plated with 500 µl of plating medium onto a DETA-covered substrate, for example a glass cover slip, and then washed with Hibernate A and some fresh plating medium. After about three cell divisions, the plating medium can be totally replaced with maintenance medium and 5 µM Roscovitine. After every four subsequent cell divisions, half of the maintenance medium can be replaced with fresh maintenance medium and 2 µM Roscovitine. The cells can be passaged and cultured under suitable conditions to establish electrical activity. One or more electrically active neurons created by the disclosed methods can then be disposed on a microelectrode array (MEA). The neural cells may be electrically active after regeneration.

Further disclosed is a MEA system, comprising a microelectrode array having a surface and at least one electrically active adult neuron disposed on a surface thereof, wherein the at least one electrically active adult neuron is from adult nervous tissue. In an aspect, the at least one electrically active adult neuron has been exposed to a cdk5 inhibitor, for example Roscovitine. In another aspect, the surface of a disclosed MEA system is treated with PDL (polynucleotide-D-Lysine) and laminin.

Disclosed is a composition comprising a population of electrically active neurons. Such a composition may be produced by a method comprising a) contacting adult neural cells with a growth factor, b) contacting the neural cells of step a) with a cdk5 inhibitor, and then c) contacting the neural cells of step b) with a neurotransmitter, wherein the adult neural cells develop into a population of electrically active neurons.

Disclosed are methods of identifying a compound that can affect electrical activity of an electrically active adult neuron, comprising a) contacting a MEA system with a test compound, wherein the MEA system comprises a microelectrode array having a surface and at least one electrically active adult neuron disposed on the surface thereof; and b) detecting a change in electrical activity of the at least one electrically active neuron or membrane portion thereof, wherein the detection of a change in electrical activity of the at least one electrically active neuron or membrane portion thereof identifies a compound that can affect electrical activity of an electrically active adult neuron. In an aspect, the electrically active adult neuron is from adult nervous tissue, for example the brain of a mammal. In another aspect, the change in electrical activity comprises changes in frequency and/or amplitude of action potentials, changes in spontaneous neuronal activity, changes in ion flow, changes in longevity, robustness (health), or protection against the presence of a toxin or other harmful or disease causing agent. Cessation of firing of an action potential by a cell indicates a change in the neural cell.

Disclosed is an automatic top-off system using a surface tension based valve, which in an example requires neither any moving parts nor electronic control devices. An example top-off system is inexpensive and convenient to miniaturize for microfluidic and/or high-throughput systems. In another aspect, the present invention comprises a liquid handling system to automatically fill wells, *e.g.,* culture wells, to desired levels without operator handling. In an examplary system, fluids are stored in a separate reservoir, and tubing is specifically allocated to each well. More particularly, a siphon system passes media from a fluid reservoir to individual wells based on surface tension of the receiving well. Liquid is passively added while a seal, such as a valve cap, remains open. After the seal is closed and initial flow is induced, the liquid media are maintained in an aspiration well at a constant level without manual manipulation. Non-limiting example applications include high-throughput cell culture and high-throughput *in vitro* drug screening. See U.S. Patent Publications 20110086427, 2009/0053749 and 20040259177 for examples of microfluidic devices that can be used in conjunction with the cells of the present invention. Implementation of the cells and such microfluidic devices can be used for drug screening. The present invention may be used in systems or combinations of microfluidic devices or a three dimensional multi-cellular surrogate tissue assembly wherein at least one component of the system or assembly comprises a population of electrically active neurons, wherein the device is used for initiating, culturing, manipulating, and assaying each tissue assembly of the system.

Another technique for drug screening provides for high throughput screening of compounds (see, *e.g.,* PCT Application WO 84/03564) that utilize robotic type systems. See, for example, U.S. Patent Publications 20100297685 and 20100105074. For example, large numbers of different small test compounds are synthesized on a solid substrate, such as plastic pins or some other surface. The test compounds are reacted with the disclosed cells, or portions thereof, of the instant application and washed. Cells are then detected by methods well known in the art, using commercially available machinery and methods, for example, the Automated Assay Optimization (AAO) software platforms (Beckman, USA) that interface with liquid handlers to enable direct statistical analysis that optimizes the assays; modular systems from CRS Robotics Corp. (Burlington, Ontario), liquid handling systems, readers, and incubators, from various companies using POLARATM (CRS), an open architecture laboratory automation software for a Ultra High Throughput Screening System; 3P (Plug & Play Peripherals) technology, which is designed to allow the user to reconfigure the automation platform by plugging in new instruments (ROBOCON, Vienna, Austria); the Allegro™ system or Staccato™ workstation (Zymark), which enables a wide range of discovery applications, including HTS, ultra HTS, and high-speed plate preparation; MICROLAB Vector software (Hamilton Co., Reno, Nev., USA) for laboratory automation programming and integration; and others.

For any of these machines and methods, the assays measure a response by the target cells that provides detectable evidence that the test compound may be efficacious. For example, the cells' response may include, but is not limited to, a change in morphology, neural processes, or synapse formation. The detectable signal is compared to control cells and the detectable signal identified by subtraction analysis. The relative abundance of the differences between the "targeted" and "untargeted" aliquots are simultaneously compared using a "subtraction" analysis (differential analysis) technique such as differential display, representational difference analysis (RDA), GEM-Gene Expression Microarrays (U.S. Pat. No. 5,545,531), suppressive subtraction hybridization (SSH) and direct sequencing (PCT Application WO 96/17957). The subtraction analysis can include the methods of differential display, representational differential analysis (RDA), suppressive subtraction hybridization (SSB), serial analysis of gene expression (SAGE), gene expression microarray (GEM), nucleic acid chip technology, or direct sequencing.

It is to be understood that this invention is not limited to specific synthetic methods, specific culture conditions, or to particular cells, as such may, of course, vary. It is also to be understood that the terminology used herein is for the purpose of describing particular embodiments only and is not intended to be limiting. Thus, reference to particular buffers, media, reagents, cells, culture conditions and the like, or to some subclass of same, is not intended to be limiting, but should be read to include all such related materials that one of ordinary skill in the art would recognize as being of interest or value in the particular context in which that discussion is presented. For example, it is often possible to substitute one buffer system or culture medium for another, such that a different but known way is used to achieve the same goals as those to which the use of a suggested method, material, or composition is directed.

All technical and scientific terms used herein, unless defined herein, are intended to have the same meaning as commonly understood by one of ordinary skill in the art. The techniques employed herein are also those that are known to one of ordinary skill in the art, unless stated otherwise.

As used in the specification and the appended claims, the singular forms "a," "an," and "the" include plural referents unless the context clearly dictates otherwise. Thus, for example, reference to "an electrically active adult neuron" can include one or a plurality of electrically active adult neurons. Reference to "a medium" includes one medium or a mixture of two or more media, and the like.

Ranges may be expressed herein as from "about" one particular value, and/or to "about" another particular value. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

In this specification and in the claims which follow, reference will be made to a number of terms which shall be defined to have the following meanings:
"Optional" or "optionally" means that the subsequently described event or circumstance may or may not occur, and that the description includes instances where said event or circumstance occurs and instances where it does not. For example, the phrase "optionally contacted before" means that contact may or may not occur before a specified event or step.

### EXAMPLES

following examples are put forth so as to provide those of ordinary skill in the art with a complete disclosure and description of how the compounds, compositions, articles, devices and/or methods claimed herein are made and evaluated, and are intended to be purely exemplary of the invention.
Efforts have been made to ensure accuracy with respect to numbers (e.g., amounts, temperature, etc.), but some errors and deviations should be accounted for. Unless indicated otherwise, parts are parts by weight, temperature is in °C or is at ambient temperature, and pressure is at or near atmospheric.

### Example 1: Conditions for culturing neurons derived from the hippocampus of adult rats:

All parameters of the adult culture system were optimized for the support of neuronal attachment, regeneration, and long-term survival *in vitro* (Figure 1.). Cellular trauma during the cell culture process was minimized by first lowering the temperature of the Dissection medium to 4°C. Next, extracellular matrix proteins and connective tissue in the brain fragments were digested using papain (2 mg / ml HA minus Ca2+, 37°C shaking water bath, 80 revolutions per minute) followed by repeated rinses of the tissue to inactivate the enzyme. Oxidative damage from free radicals released during tissue dissociation was limited by inclusion of the powerful anti-oxidants Trolox (D. Mulkey et al., 2003) and dextrose-coated cerium oxide nanoparticles (M. Das et al., 2007) in the Dissociation and Plating medium. Direct neuro-protection against apoptotic agents was achieved by adding caspase inhibitors (1, 3, and 6) to the Dissociation medium. The tissue was dissociated with fire-polished glass Pasteur pipettes in order to maximize cell breakup while minimizing cellular trauma. Last, applying the dissociated neurons onto glass cover slips coated with the cell adhesive surface DETA in Plating medium for 45 minutes allowed adherent neurons to attach with minimal attachment of cellular debris. Following this plating period, a gentle wash of the cover slips with warm HA (37°C) caused the majority of cellular debris, including myelin inhibitory factors present after the breakup of myelin sheaths (Y. Z. Alabed et al., 2006), to be removed. Improvements to these basic culture parameters optimized neuronal survival and regeneration (Figures 2-3), allowing for investigation into the electrical properties and expression patterns displayed by adult neurons in a controlled *in vitro* environment (Figure 4).

### Example 2: Inhibition of cell cycle progression in mature neurons in vitro

Media formulations along with the time- and dose-dependent application of growth factor and transcription factor mediators (Figure 1) were applied to promote neuronal recovery and long-term functional survival. While these improved culture system parameters supported adult neurons, they also forced these terminally differentiated primary neurons to enter the cell cycle and divide. While critical growth factors, specifically basic fibroblast growth factor (bFGF) and dissociated cell culture conditions supported the survival and regeneration of mature neurons derived from adult rat hippocampal tissue, they also triggered re-entry into the cell cycle and division through up-regulation of cyclin and cyclin-dependent kinase (cdk) expression (S. B. Rodan et al., 1989; H. Katsuki et al., 2000; L. Munaron, 2002; S. M. Goodyear and M. C. Sharma, 2005; S. Goodyear and M. C. Sharma, 2007). The mitotic division of these neurons was arrested *in vitro* after neurons reached confluency (Figure 2B, control) and these previously dividing neurons were shown to exhibit both morphological and electrical recovery after 25 µM glutamate was added to the culture media for at least 24 hours (D. Edwards et al., 2010). Despite the utility of this system, for more relevant investigations into primary dissociated neurons, the rapid proliferation of neurons must be eliminated.

Multiple strategies were investigated to prevent primary terminally differentiated adult hippocampal neurons from returning to the cell cycle and dividing. The ultimately successful strategy for preventing proliferation under cell culture conditions was to add roscovitine, a competitive cdk inhibitor. Roscovitine was introduced into the culture medium at 1, 5, and 10 µM both before the onset of neuronal division (2 DIV) and after the onset of neuronal division (7 DIV) (Figures 2A, B). When 1 µM roscovitine was applied on day 2, the cell population increased more slowly than without roscovitine (Figure 2A). 10 µM roscovitine caused a decrease in the neuronal population when applied on day 2, and a sharp loss of neurons when introduced on day 7 (Figure 2B). A stable population of neurons was achieved through the application of 5 µM roscovitine on days 2-7 *in vitro* and 2 µM after day 7 as a long-term "booster" concentration (Figure 2B). In addition, the health and maturity of this stable neuronal population was evident in the rapid and lengthy extension of primary axons by these neurons, to as much as 4-5 times longer on average versus untreated control neurons (Figure 2C). A stable population of neurons derived from the hippocampus of adult rats was achieved using this cell culture system and roscovitine treatment. Where control neuron populations proliferated to confluency, roscovitine treated neuronal populations did not. These roscovitine treated, non-proliferating cultured neurons exhibited morphological characteristics previously seen exclusively in cultured embryonic neurons. Additionally, these non-proliferating neurons expressed the mature neuronal markers MAP2 and NeuN, but lacked expression for the nuclear marker of proliferating cells, Ki-67 After several weeks in culture, the population of neurons *in vitro* remained stable (Figures 2A, B), with very few neurons detaching from the DETA surfaces.

Other strategies for mitotic elimination that ultimately proved ineffective in the prevention of neuronal proliferation *in vitro* were also investigated. First, bFGF was removed from the Plating media and/or the Maintenance media. Neurons exposed to medium lacking bFGF did not divide. Rather than remaining static, however, neuronal populations deprived of bFGF decreased rapidly *in vitro,* with almost complete loss of neurons evident after two weeks (Figure 2A: FGF-P AND FGF-M, 4A). During this two week period, the morphology of the neurons dramatically changed, with the axon and branched dendrites retracting toward the cell body and ultimately forming a branched ring of neurites that surrounded the cell soma The factor bFGF proved to be an essential component of the serum-free culture medium for support of neuronal survival, recovery, and regeneration.

Mitotic inhibitors were also investigated to eliminate the division of the cultured neurons. FudR (10 and 50 µM) and ara-C were added to the culture medium on 2 DIV (Figure 2A: F-10, F-50, and ara-C). This strategy was unsuccessful because all dividing cells were forced into apoptosis, eliminating the cultured primary neurons rather than preventing neuronal division. Additional new agents, Trolox and Aphidicolin, both with potential anti-mitotic activity, were tested to inhibit the division of neurons in this culture system. Trolox, an antioxidant with anti-mitotic properties toward cancerous human breast cancer cells (A. e. a. Charpentier, 1993), was not effective in preventing primary neurons from returning to cycle *in vitro* at both 40 and 100µM (Figure 2A: T-40, T-100). Its antioxidant activity was found, however, to improve neuronal survival (Figure 2A), and was incorporated into both the Dissociation and Plating media at 70nM (Table 1). Aphidicolin, a cell cycle inhibitor previously effective against bone marrow cell division (D. S. Dimitrova and D. M. Gilbert, 2000), also did not display effective anti-mitotic activity. At 1.5 µM, a concentration high enough to impede the return of the neurons to the cell cycle, aphidicolin caused most cultured neurons to either necrose or apoptose (Figure 2A: A-1.5), and limited regeneration of neurites and functional electrical properties of those surviving neurons.

### Example 3: Electrical activity and membrane channel expression of adult hippocampal neurons in vitro after elimination of mitotic activity

Neurons were evaluated for electrical recovery after 6, 13, and 25 DIV using whole-cell patch clamp electrophysiology to evaluate the electrical potential of neurons, defined by the ability to move current into and out of the cell and to fire and propagate action potentials. The recovery of electrical activity *in vitro* was found to be significantly different between populations of neurons allowed to divide unchecked and neurons where mitotic division was prevented through application of roscovitine. When allowed to mitotically divide unchecked *in vitro,* neurons did not fully recover electrically for up to 3 weeks *in vitro* and then only after the neurons reached confluency and were stimulated with 25 µM glutamate for more than 24 hours (D. Edwards et al., 2010). Prevention of neuronal division *in vitro* through application of roscovitine (5 µM days 2-7 *in vitro* followed by 2 µM roscovitine after day 7) significantly changed the time-scale required for electrical recovery of neurons *in vitro.* Glutamate (25 µM) had been applied after 5 DIV. Neurons recovered after 6 DIV and showed the ability to move current into and out of the cell as well as to fire action potentials (Figure 4A, column 1). Further recovery of these neurons was found after 13 and 25 DIV, as seen in increased resting membrane potential (Vm), increased membrane resistance (Rm), increased current flow, both inward sodium and outward potassium, as well as increased action potential amplitude (Figure 4, A). Figure 4B shows example traces displaying current flow into and out the neuron and an action potential.

### Example 4: Protein expression indicates maturity of adult hippocampal neurons in vitro

The maturity of neurons cultured in this system was probed. Neurons that were allowed to mitotically proliferate were probed for expression of neurofilament-M, Ki-67, and NeuN. While positive for the neuron-specific structural protein neurofilament-M, none of the neurons was positive for the mature neuronal nuclear marker NeuN. Instead, all neurons were positive for Ki-67, expressed exclusively in dividing neurons active in the cell cycle. After roscovitine was added to the cell culture medium, neurons no longer divided, and the expression pattern changed. Neurons still expressed mature structural proteins (MAP2), but instead of the proliferation marker Ki-67, they expressed the mature nuclear marker NeuN (Figure 2E). These neurons were also probed for NMDA channel subunit expression. Neurons in this cell culture system were probed immunocytochemically for each of the two subunits, and NR2A subunits outnumbered NR2B subunits, with both subunits present.

### Example 5: Expansion of a population of primary neurons in vitro

The combination of bFGF and roscovitine has allowed for a culture system of terminally
d primary adult hippocampal neurons in which the neuronal population can be kept stable or can be expanded. When these neurons were expanded under the basic culture conditions described in Figure 1, *i.e.* without roscovitine, three distinct phases were observed. In phase 1 (0-3 DIV), mature neurons were able to recover from the trauma of cell culture and regenerated morphologically, as shown by the expression of neurofilamant-M, the 150 kDa structural protein uniquely found in neurons in the brain, and NeuN, a nuclear marker expressed exclusively in mature neurons (R. J. Mullen et al., 1992). The components of the Plating medium
, specifically growth factors, anti-oxidants, and Neurobasal-A with adjusted osmolarity, were essential for this recovery and regeneration. In phase 2 (3-21 DIV), bFGF in the Maintenance media induced neurons to re-enter the cell cycle through its activity in up regulating cdk5 expression (B.-S. Li et al., 2001; Z. Yan et al., 2002; S. M. Goodyear and M. C. Sharma, 2005; B. Menn et al., 2010). Previously quiescent, terminally differentiated phase bright neurons with long axons and branched dendritic trees first retracted their neurites into the cell soma, divided, and finally re-extended their axons and dendrites a period observed to occur on average 90 minutes from neurite retraction, division, and neurite extension. Neurons that have previously divided repeat this process of cell division and double in number on average every 24-36 hours. During these periods, neurons displayed expression patterns consistent with both neurons and dividing cells, with all cells displaying both neuronal morphology and expression of Ki-67, a nuclear marker for proliferating cells (T. Scholzen and J. Gerdes, 2000), and Nf-M. At the same time, however, these dividing neurons were not expressing NeuN. Based on the pattern of all neurons during phase 1, specifically with all cells expressing mature neuronal markers, as well as the universal expression of proliferation markers throughout phase 2, it was determined that mature neurons were dividing, not the subset of neural progenitors known to exist in the dentate gyrus of the hippocampus. If only amplification of the smaller progenitor neuronal population of neurons had been occurring, then only a subset of neurons present would have shown evidence of Ki-67 expression.

In phase 3 (21-90 DIV), neurons reached confluence and stopped proliferating, a point at which neuronal expression of Ki-67 ended and NeuN reappeared. However, a passage of the confluent neurons from one DETA cover slip to multiple DETA cover slips at a lower cell density allowed for the continued proliferation of neurons beyond that seen on one cover slip alone, extending phase 2. During phase 2 neuronal electrical activity, specifically the ability to move current into and out of the cell and to fire action potentials (APs) was limited or non-existent. As the cells moved into phase 3, neurons were stimulated to recover their electrical activity through the introduction of 25 µM glutamate to the Maintenance medium. When cycloheximide (CHX), an inhibitor of protein synthesis that had previously been used to block preconditioning in neurons (F. C. Barone et al., 1998), was used to block all possible changes in protein synthesis induced by incubation of the neurons with glutamate, all improvements in neuronal electrical activity were completely blocked. The introduction of glutamate mediated the cellular transcription activity to mature the previously dividing neurons (D. Edwards et al., 2010).

### Materials and Methods for Examples 1-5

### DETA surface modification and characterization

Glass cover slips (Thomas Scientific 6661F52, 22mm×22mm no. 1) were cleaned by acid washing using a 50/50 mixture of concentrated hydrochloric acid and methanol. The cover slips were washed three times, 30 min per wash, and were rinsed in distilled de-ionized water between each washing. The DETA (N-1 [3-(trimethoxysilyl) propyl]-diethylenetriamine, United Chemical Technologies Inc., Bristol, PA, T2910KG) monolayer was formed by the reaction of the cleaned surface with a 0.1 % (v/v) mixture of the organosilane in freshly distilled toluene (Fisher T2904) (M. Ravenscroft et al., 1998). The DETA-coated cover slips were heated to just below the boiling point of toluene, rinsed with toluene, reheated to just below the boiling temperature, and then oven dried. The DETA formed a reaction site limited monolayer on the surface of the cover slip (M. Ravenscroft et al., 1998). The DETA cover slips were characterized to authenticate the monolayer formation. First, contact angle measurements were taken using an optical contact angle goniometer (KSV Instruments, Monroe, CT, Cam 200). The contact angle for the DETA-coated cover slips was 54.2±0.2, which was previously shown to be acceptable for neuronal hippocampal culture (M. Ravenscroft et al., 1998). Second, X-ray Photoelectron Spectroscopy (XPS) (FISONS ESCALab 220i-XL) was used to characterize the elemental and chemical state of the DETA-coated cover slip surfaces. The XPS survey scans as well as high-resolution N 1s and C 1s scans, using monochromatic A1 Kα excitation, were obtained, similar to previously reported results (M. Ravenscroft et al., 1998; M. Das et al., 2005).

### Cell culture methodology and medium used for adult rat hippocampal culture

The hippocampi of adult rats (Charles River, age 6-12 months) were dissected and homogenized into small tissue fragments in cold medium (∼4°C) consisting of Hibernate-A,
X, and antibiotic-antimycotic. The tissue was then digested for 30 minutes at 37°C in calcium-free Hibernate-A (HA) containing 6 mg papain / 12 ml (HA). Following digestion, the tissue was washed three times with cold Hibernate-A media to remove any active enzyme. Next, the tissue was suspended in Dissociation Media , formulated for dissociation of rat and mouse adult brain tissue into individual cells and dissociated into individual cells through mechanical dissociation with fire-polished Pasteur pipettes. The dissociated cells were suspended in Plating Media , used after tissue dissociation to promote neuronal attachment and regeneration, and then deposited onto DETA-coated glass cover slips for 30-45 minutes. DETA, with its triamine functional group exposed at the surface, strongly attaches to neurons and allows for all non-neuronal debris such as ECM proteins, myelin debris, and cell fragments to be washed from the cover slip surface (D. A. Stenger et al., 1993; J. J. Hickman et al., 1994; M. Das et al., 2005; A. E. Schaffner et al., 1995). Following this washing step, fresh Plating medium was applied and remained for the first 3 DIV. On 3 DIV and every fourth subsequent day, one half of the medium was removed and replaced with Maintenance Media (Figure 1), supporting the long-term maintenance of these neurons. Each type of medium was formulated to specifically meet the challenges presented during each stage of the cell culture process, allowing for significantly improved survival, regeneration, and long-term growth ( Figure 1). The osmolarity of the media was adjusted to match the osmolarity of adult rat cerebrospinal fluid (295-305mOsm) (S. C. Baraban et al., 1997; R. A. Fishman and G. D. Silverberg, 2002). In the Dissociation Medium the use of caspase inhibitors and antioxidants (M. Das et al., 2007) during the dissociation of the tissue minimized both oxidative damage as well as the progression into apoptosis which normally results from cellular damage during this stage. Inclusion of these antioxidants as well as specific growth factors in the Plating Media was found to promote attachment and regeneration of neurons *in vitro.* For long-term survival, the anti-oxidants were removed from the Maintenance Medium.

### Control of Neuronal Division in vitro

bFGF (Invitrogen, 13256-029, 5ng/ml) triggered neuronal cell proliferation until confluence, a period marked both by rapid neuronal division as well as limited or absent functional electrical activity. After neuronal confluence, 25 µM glutamate ((N-Acetyl-L-glutamic acid, Aldrich, 855642) triggers induction of neuronal electrical recovery (D. Edwards et al., 2010). Control of the division of neurons in this culture system was investigated experimentally as follows: (1) bFGF removal from both the Plating and Maintenance medium. (2) Fluorodeoxyuridine (FudR, Sigma-Aldrich, F-0503) addition at 10 and 50 µM (3) arabinoside-D (ara-C, Sigma, C-6645) plus deoxycytidine addition. (4) Trolox (Sigma, 238813) addition at 40 and 100 µM. (5) Aphidicolin (Aph, Sigma, A0781) addition at 1.5 µM. (6) Roscovitine (Rosc, Sigma, R7772) addition at 1, 5, and 10 µM. All factors were added before the onset of neuronal division (2 DIV), some after the onset of division (after 3 DIV).

### Electrophysiology

Whole-cell, patch-clamp recordings were performed at room temperature. pH was adjusted to 7.3 with HEPES acid and base, ion concentrations with NaCl to approximately 130 mM, and osmolarity of 290-300 mOsm. Cells were visualized on the stage with a Zeiss Axioscope, 2 FS Plus, upright microscope in maintenance culture medium. Patch pipettes (4-8 M'Ω) were filled with intracellular solution (K-gluconate 140 mM, EGTA 1 mM, MgCl₂ 2 mM, Na₂ATP 5 mM, HEPES 10 mM; pH 7.2), osmolarity approximately 260 mOsm. Voltage clamp and current clamp experiments were performed with a Multiclamp 700A (MDS Analytical Devices) amplifier. Signals were filtered at 3 kHz and digitized at 20 kHz with an Axon Digidata 1322A interface. Data recordings and analysis were performed with pClamp software. Whole-cell capacitance and series resistance were compensated electronically. Only cells with access resistance less than 22 M'Ω were analyzed. Inward currents that had the characteristics of fast sodium currents, and outward currents that had the characteristics of potassium currents were measured in voltage clamp mode using voltage steps of 10 mV from a -70 mV holding potential. Action potentials were measured with 1 s depolarizing current injections to elicit action potentials from a -70 mV holding potential. Cells were morphologically selected for electrophysiological characterization. Selected cells were phase bright pyramidal neurons with large branching apical dendrites and small basal dendrites. Cells with this morphology stained positive for anti-neurofilament-M (Nf-M) and/or MAP2. Neurons were electrically characterized after 6, 13, and 25 DIV.

### Immunocytochemistry

To prepare cells for immunocytochemical characterization, cover slips were rinsed twice with Phosphate Buffered Saline (PBS). Cells were fixed with 4% paraformaldehyde for ten minutes at room temperature, and subsequently rinsed three times with PBS. Cells were permeabilized for five min in PBS with 5 mM Lysine and 0.5% Triton X-100, and were then blocked for two hours by adding 5% normal donkey serum. Anti-Neurofilament-M polyclonal antibody (Nf-M, intracellular filament found in mature neurons, Chemicon, AB 1981, diluted 1:1000), anti-NeuN (nuclear marker in mature neurons, Chemicon, diluted 1:1000), anti-Ki-67 (nuclear marker in dividing cells, Chemicon, diluted 1:1000), anti-NR2A (NMDA channel subunit expressed in the post-synaptic cell membrane, AB1548, diluted 1:75), anti-NR2B (NMDA channel subunit expressed in the post-synaptic cell membrane, MAB5216, diluted 1:150) were added in blocking solution for 12 hr at 4°C. After 3 washes with PBS, fluorescently labeled secondary antibodies (Invitrogen, A11011, A21449, and A11029) were applied for two hours. Vectashield mounting medium (H1000, Vector Laboratories, Burlingame, CA) was used to mount the cover slips onto slides. The cover slips were observed with an Ultra VIEWTM LCI confocal imaging system (Perkin Elmer).

### Statistical analysis

Numerical summary results are reported as a mean, plus or minus the sample standard error of the mean (± SEM). Neuronal cell density immediately following plating onto cover slips was normalized to 100 to allow for comparisons of the effect of growth factor removal or mitotic factors intervention. For two-group comparisons, statistical analysis included a two-sample student's t-test on electrophysiological data.

### Example 6: Manipulation of the cell cycle in mature terminally differentiated neurons to create an expandable neuronal cell line:

Expansion of a population of mature terminally differentiated neurons derived from the hippocampus of adult rats, passage of that population 4 times, and arresting proliferation in ½ the population after each passage followed by electrical characterization

Method: Mature terminally differentiated neurons were extracted from the hippocampus of adult rats and plated onto DETA cover slips. bFGF in the culture medium caused these neurons to re-enter the cell cycle and proliferate to confluency on the cover slip. The following passaging protocol was used

### Cell Passaging Technique (in brief):

1. Initial culture - two adult rats plated for 1 hour. Post hour wash to remove myelin debris and addition of Plating medium
2. Cell confluent on cover slip(s) (approximately 14-23 days *in vitro*)- initial passage
   a. Cover slip(s) trypsinized with .05% trypsin / EDTA in HBSS
   b. Upon initial cell dissociation, trypsin inhibitor added
   c. Cells washed off surface, removed to 15mL flask and spun at 500Xg for 5 mins
   d. X 2 more washes before replating upon fresh DETA coverslips
      ½ cover slip(s): proliferation arrested with cdk inhibitor roscovitine followed by electrical characterization through electrophysiology
      ½ cover slip(s): continued proliferation followed by passaging
3. Subsequent passages. Cells adhere and recover / proliferate to confluency
4. After confluency, neurons dissociated and replated on fresh DETA coverslips and/or micro-electrode arrays.

### Example 7: Adult Neuronal Function and Synaptic Activity in a High-Throughput MEA System: Differential expression patterns, synaptic connections, network communication, and response to drug and therapeutic intervention between neurons derived embryonic or adult brains.

### Microel

experiments for the study of synaptic activity and neural connectivity in populations of neurons from dissociated cultures. Uses for such an *in-vitro* high-throughput test system include investigations into neurodegenerative disease, traumatic brain injury, and stroke. A common limitation of neuronal MEA systems has been the reliance upon neurons derived from embryonic tissue. With MEAs, spontaneous network activity from both embryonic and adult brain tissue was concurrently measured. In addition, introduction of agonists and antagonists that affect maturation of neuronal activity/synaptic transmission established the usefulness of this system for drug discovery and basic research, including neuronal development and regeneration.

### Method:

Adult neurons: Mature terminally differentiated neurons were extracted from the hippocampus of adult rats and plated onto DETA cover slips. After 4 days the neurons were passaged from these cover slips onto MEAs that had been coated with PDL / laminin (for cell adhesion)

Embryonic neurons: Neurons from the hippocampus of embryonic day 18 rat fetuses were extracted and plated directly on MEAs that had been coated with PDL / laminin..

For each population of neurons, electrical recordings of spontaneous neuronal activity were performed for up to 3 months. In addition, antagonists and agonists were introduced and their effects were measured against baseline electrical activity. 64-channel axion MEAs were used.

Results: Neurons (both embryonic and adult) regenerated on the MEAs and recovered electrically. After two weeks on the MEAs, both populations demonstrated consistently stable electrical activity.

### Example 8: Embryonic rat hippocampal dissociated cell culture methodology

Embryonic hippocampal neurons were cultured. Pregnant rats, 18 days in gestation, obtained from Charles River were euthanized with carbon dioxide and the fetuses were collected in ice-cold Hibernate E (BrainBits)/B27/Glutamaxtm/Antibiotic-antimycotic (Invitrogen) (Dissecting Medium). Each fetus was decapitated, and the whole brain was transferred to fresh ice cold dissecting medium. After isolation, the hippocampi were collected in a fresh tube of dissecting medium. The tissue was enzymatically digested at 37°C for 10 minutes with papain (Worthington 3119), 12 mg / 6 ml Hibernate-E (- calcium) (BrainBits) + 0.5 mM Glutamax (Invitrogen). Hippocampal neurons were obtained by triturating the tissue using a fire-polished Pasteur pipette. After centrifugation, the cells were re-suspended in culture medium (Neurobasal/B27/Glutamaxtm/antibiotic-antimycotic) and plated on MEAs. All research was approved by the Institutional Animal Care and Use Committee at the University of Central Florida and conformed to NIH guidelines.

### Adult rat hippocampal dissociated cell culture methodology

Adult neurons were extracted, dissociated, cultured, and maintained. Briefly, the hippocampi of adult rats (Charles River, age 6-12 months) were dissected and homogenized into small tissue fragments in cold medium (∼4°C) consisting of Hibernate-A, glutamax, and antibiotic-antimycotic. The tissue was digested for 30 minutes at 37°C in calcium-free Hibernate-A (HA) containing 6 mg papain / 12 ml (HA no calcium). Following digestion, the tissue was washed three times with cold HA media to remove any active enzyme. Next, the tissue was suspended in Dissociation Medium and broken apart into individual cells through mechanical dissociation with fire-polished Pasteur pipettes. The dissociated cells were suspended in Plating medium and then deposited onto DETA (N-1 [3-(trimethoxysilyl) propyl]-diethylenetriamine, United Chemical Technologies Inc., Bristol, PA, T2910KG) coated glass cover slips. After 30-45 minutes, the cover slips were washed with warm HA by gently swirling the medium to remove tissue debris. Following this washing step, fresh Plating medium was applied and remained for the first 3 div. On 3 div the medium was removed and replaced by Maintenance Medium with 5 µM Roscovitine (Rosc, Sigma, R7772). All research was approved by the Institutional Animal Care and Use Committee at the University of Central Florida and conformed to NIH guidelines. After 4 div, the adult hippocampal neurons on the DETA cover slips were passaged to MEAs. Briefly, neurons were dislodged from the DETA with trypsin (0.05% trypsin / EDTA in HBSS, Gibco, 25200). Trypsin inhibitor (trypsin inhibitor, soybean, Gibco, 17075-029) in Dissociation medium at 0.5 mg per ml deactivated the trypsin. The dislodged neurons were collected and spun at 500 x g for 5 minutes. The supernatant of deactivated trypsin in HBSS was discarded, and the neuronal cell pellet was suspended in 1 ml Plating medium.

### Immunocytochemistry and Laser Scanning Confocal Microscopy

To prepare cells for immunocytochemical characterization, cover slips were rinsed twice with Phosphate Buffered Saline (PBS). Cells were fixed with 4% paraformaldehyde for ten minutes at room temperature, and subsequently rinsed three times with PBS. Cells were permeabilized for five minutes with 0.5% Triton X-100 in PBS, and were then blocked for two hours in 5% normal goat serum in PBS. Anti-neurofilament-M (Chemicon, AB5735, 1:500), anti-synaptophysin (Chemicon, MAB368, 1:300), and either anti-NMDAR2A (Chemicon, AB1555P, 1:200), anti-NMDAR2B (Chemicon, AB15557P, 1:200), or anti-glutamate receptor 2/3 (Chemicon, AB1506, 1:50) were added in blocking solution for 12 hr at 4°C. After 3 washes with PBS, fluorescently labeled secondary antibodies (Invitrogen, A11011 (594nm), A21449 (647nm), and A11029 (488nm), 1:200) in blocking buffer were applied for two hours. Vectashield mounting medium with DAPI (H1200, Vector Laboratories, Burlingame, CA) was used to mount the cover slips onto slides. Fluorescent images were acquired with the UltraView spinning disc confocal system (PerkinElmer) with AxioObserver.Z 1 (Carl Zeiss) stand, and a Plan-Apochromat 40×/1.4 Oil DIC plan-apochromat objective with 26 µm resolution. Z-stack projections of the scanned images were generated and modified within the Volocity image processing program (PerkinElmer).

### Extracellular recordings

The MEA chips (Axion Biosystems) provided 64 platinum-black coated gold-electrodes with a diameter of 30 µm, organized in an 8 by 8 array with 200 µm pitch. Clean MEAs were sterilized with 70% alcohol and then incubated with 1 ml poly-L-lysine (100 µg/ml) for 30 minutes. An area just large enough to cover all electrodes was additionally coated with 3 µl laminin (2 µg/ml) over night. Embryonic rat hippocampal neurons were plated directly on MEAs at a density of 500 cells/mm2. Adult rat hippocampal neurons were first cultured on DETA coated cover slips for recovery. While basic fibroblast growth factor (bFGF) was necessary for survival of adult neurons in culture, bFGF caused rat neurons to divide *in vitro;* administration of Roscovitine at 3 div prevented neuronal mitotic activity. After 4 days on DETA cover slips, the adult neurons were passaged onto MEAs at a density of 500 cells/mm2. Every 2-3 days, half the medium was replaced with fresh Maintenance medium supplemented with 2 µM Rosc. Supplementing the adult neurons with 25 µM glutamate (N-Acetyl-L-glutamic acid, Aldrich, 855642) on the 2 div increased the electrical activity of the adult neurons.

MEAs were incubated with their covers off to allow gas exchange but covered upon removal from the incubator to reduce contamination, media evaporation, and gas exchange while recordings were taken. The head stage of the recording system (Axion Biosystems) was pre-heated to 37 °C before MEAs with adult or embryonic hippocampal cultures were investigated. The activity of neuronal networks was recorded with 25 kHz using the software Axion's Integrated Studio (AxIS). Signal amplitudes 6 times larger than the standard deviation of the base line were detected as action-potential spikes. The spike data was then imported into Matlab 201 Ob (The MathWorks) for further processing.

### Experimental Procedure

Baseline spontaneous activity in adult and embryonic neurons was recorded for 3 minutes on 5 consecutive days per week, starting on 7 div. The synaptic antagonists D-(-)-2-Amino-5-phosphonopentanoic acid (D-AP5, 25µM, Tocris Bioscience, 0106), 6-Cyano-7-nitroquinoxaline-2,3-dione disodium (CNQX, 25µM, Tocris Bioscience, 1045), and (-)-Bicuculline methobromide (Bicuculline, 50µM, Tocris Bioscience, 0109) were separately administered to both adult and embryonic neurons on 14 div and at various time points between 30 and 60 div. Three minute recordings were made to quantify the effect from these antagonists on spontaneous activity. If necessary, additional baselines were recorded prior to the administration of antagonists. Each experiment was followed by a complete washout of the antagonist and replacement with fresh medium.

### Evaluation and statistics

Data analysis was performed off-line using unpublished Matlab functions to evaluate spike files created during each MEA recording. In brief, each 3 minute dataset was processed to extract the following parameters: "active channels" (a number from 0 to 64); "AP frequencies" (reciprocal inter-spike intervals); "Burst" (where at least 10% of the active channels showed more than 1 AP with 1 ms); "Burst duration" (time-interval from the first AP in a burst to the last AP in a burst); "Average burst frequency" (the reciprocal time-difference between two subsequent bursts); "In-burst frequency" (amount of APs within a detected burst divided by the duration of that burst); "Non-burst frequency" (reciprocal inter-spike intervals between APs that were not associated with bursts). In about 25% of the adult cultures and 14% of the embryonic cultures, portions of the neuronal networks detached from the substrate over time and especially after about 6 weeks. In order to accommodate the different numbers of active channels between MEAs, all values were corrected for the amount of active channels on an MEA. For each of the 3 minute recordings, a representative results chart was produced as shown inFigures 6A-6D. Effects from synaptic antagonists were measured as the percent of active channels or AP frequency of treated embryonic or adult cultures versus the baseline activity of the same cultures recorded before administration of the antagonist.

### Results

Dissociated neuronal cultures from adult and embryonic sources recovered on MEAs and formed networks. Three-minute recordings of spontaneous activity were taken of each MEA, 5 times per week, for up to 70 div. Phase-contrast pictures of the MEAs were taken after each recording for a daily assessment into the condition of the cells as well as verification of physical contact between cells and electrodes

### Spontaneous activity of Adult and Embryonic Neurons

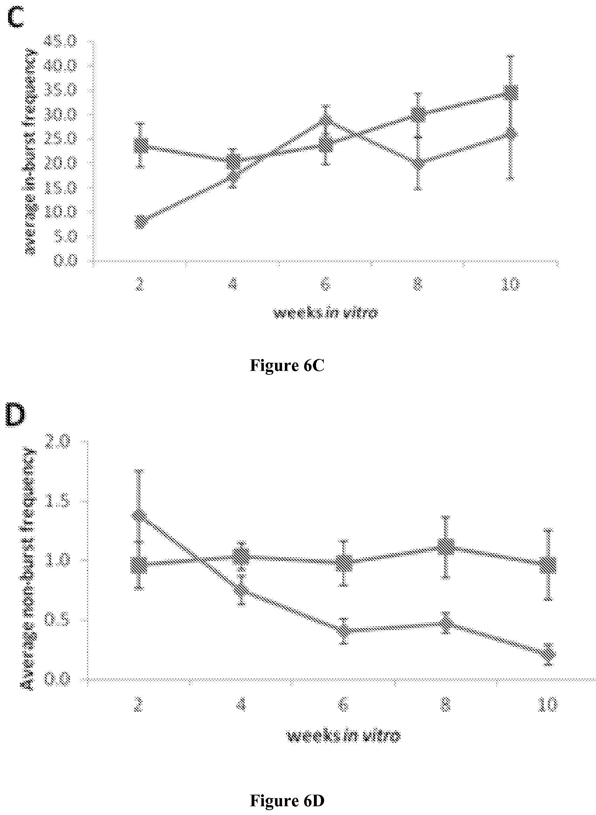

Spontaneous firing activity started in both the adult and embryonic neurons between 7-10 div. Movement of MEAs from the incubator to the heated recording stage and the subsequent 3 minute recording period did not significantly affect the pH or temperature of the medium, represented by consistent baseline activity. However, brief increases in baseline neuronal activity were observed as a result of medium changes. Over the more than 2 month culture period, embryonic MEAs (n = 6) consistently displayed a higher number of active channels, with an average of 37±8 channels active per MEA versus 15±5 channels in adult MEA cultures (n = 9). Action potential (AP) firing frequencies in embryonic cultures with approximately 2 - 4 Hz were higher as in adult cultures with 1 - 2 Hz. Spontaneous bursting occurred about one week earlier in adult cultures as opposed to embryonic cultures. The burst development over the first 6 weeks was consistent between the two types of neuronal networks (Figure 6A). After the 6th week, however, bursts in the adult cultures appeared less often, whereas the burst frequency in the embryonic cultures increased further. For both, the embryonic and adult cultures, the duration of bursts decreased over the time span of 10 weeks (Figure 6B). The bursts of embryonic cultures were in average 3 - 5 times longer as opposed to bursts in adult cultures. While burst durations in the embryonic cultures stayed very variable (about ±1s) over the 10 weeks of experimentation, bursts in the adult cultures decreased in length. This focusing of bursts in adult cultures was accompanied by a steady increase of the in-burst firing frequencies over time (Figure 6C). The in-burst frequencies of adult cultures recovered within the first 2 weeks, whereas embryonic cultures recovered slower and reached mature in-burst levels after about 6 weeks. The non-burst firing frequencies (APs not related with bursts) were consistent over the entire time for the adult cultures, but decreased steadily in the embryonic cultures (Figure 6D). Overall, the burst activity focused and matured earlier and more consisted in adult cultures on MEAs, whereas embryonic cultures showed a slower and more chaotic maturation.

### The effect of synaptic antagonists on the activity of adult and embryonic neurons

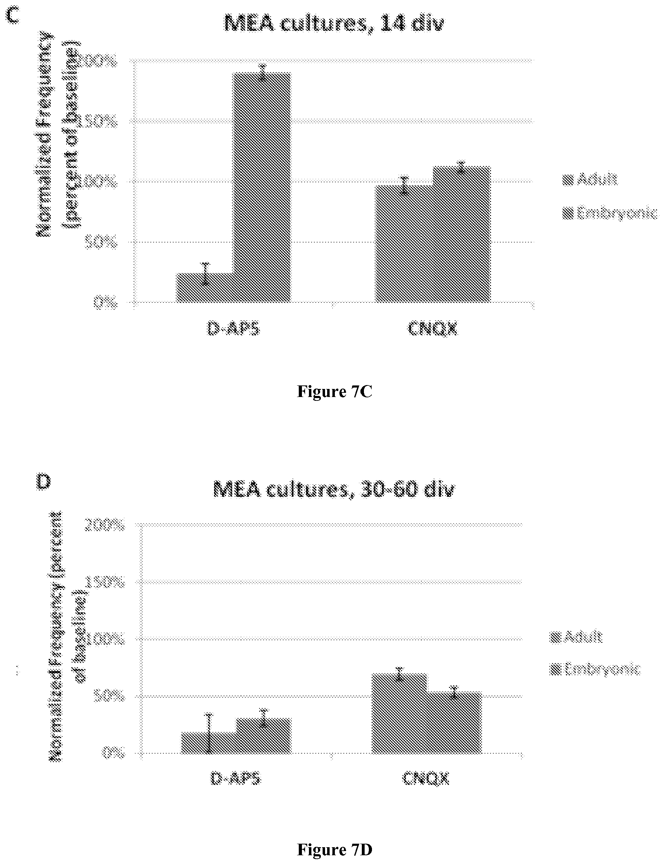

Agonist and antagonist were administered to embryonic and adult cultures on MEAs in separate experiments: 25 µM D-AP5 (NMDA channel antagonist), 25 µM CNQX (AMPA channel antagonist), and 50 µM bicuculline (GABAA antagonist). The results were summarized in Figure 7.

D-AP5 (25 µM) caused a significant decrease in the number of active channels in both adult and embryonic MEA cultures. Adult cultures lost a greater percentage of channel activity (90±6 % on 14 div, 82±6 % on 30-60 div) versus embryonic cultures (65±4 % on 14 div, 36±7 % on 30-60 div). Changes in the action potential frequency also varied with lower frequencies in adult cultures (76±8 % on 14 div, 82±17 % on 30-60 div) which were significantly different from the measured effect on embryonic cultures. On 14 div, the firing rate of embryonic neurons increased 90±6 %, while in 30-60 day old cultures the firing rate decreased 70±7 %. The difference in NMDA channel expression (NR2A and NR2B subunits) in adult and embryonic neurons *in vitro* likely caused the contrasting reaction to D-AP5 in the two populations of neurons. While NMDA channels were expressed at low levels in embryonic neurons on 14 div, the level of expression was not nearly as high as in adult neurons on 14 div. Because adult neurons expressed a greater number of NMDA channels, their reaction to the competitive NMDA antagonist D-AP5 was much more pronounced.

Addition of CNQX caused the activity of far fewer channels to be lost in both adult and embryonic MEA cultures as compared to D-AP5 (Figure 7). Adult cultures lost a greater percentage of channel activity (52±3 % on 14 div, 24±5 % on 30-60 div) versus embryonic cultures (23±5% on 14 div, 0±7% on 30-60 div). Changes in the action potential frequency in both the adult and embryonic cultures were not affected by CNQX after 14 div. The activity of the neurons was only slighted decreased in both embryonic and adult cultures between 30 to 60 div (31±6% drop in adult MEAs, 47±5% drop in embryonic MEAs). While expression of the AMPA channel subunits GluR2/3 was not observed in embryonic neurons on 2 div, expression had increased to mirror adult levels by 14 div

After MEA measurements were recorded, the antagonists were washed from the neurons with an entire media change. 24 hours after, the activity of the neurons had returned to baseline levels.

### Results

Adult neurons cultured from the hippocampus of rats recovered functionally and had the capacity to fire spontaneously on MEAs over 70 div. These studies of neuronal maturation highlight the need for gene and protein expression in study populations of neurons to mirror that in mature adult neurons *in vivo.* Because embryonic neurons do not express the same machinery responsible for electrical activity or signal propagation in adult neurons, then their response to neurotoxic agents or drug therapies may not be correlative to responses in the mature adult brain.

The disclosed method of culturing adult neurons resulted in a system where NMDA and AMPA channel subunits were expressed throughout the lifespan of the culture. NMDA channel subunits NR2A and NR2B, as well as AMPA channel subunits GluR2/3, were expressed on and after 2 div. This contrasted greatly from neurons derived from embryonic tissue, with delayed NMDA and AMPA channel expression until 14 div and later. Responses of neurons to NMDA and AMPA channel antagonists were found to be significantly different in embryonic neurons as compared to adult neurons, with each antagonist decreasing activity in adult neurons to a greater degree than in embryonic neurons. These results show that embryonic neurons in culture develop a mature profile of ion channel subunits after 3-4 weeks. Therefore, embryonic neurons should not be employed for further experiments until they have fully matured in culture especially in studying neurodegenerative diseases such as Alzheimer's where synaptic protein profiles may play a critical role in the process of synaptic failure.

In comparison to embryonic MEA systems, as disclosed herein, measurements of neuronal activity using adult hippocampal neurons on MEAs, is more applicable to the adult brain. While preparation of these MEAs was slightly more complicated than embryonic neuronal MEAs, the end result yielded a long-term screen methodology that is more correlative to the dynamics of learning and memory formation in the adult brain. Additionally, due to the earlier development of bursts and general maturation, this system can facilitate quicker, more reliable, and more correlative investigations into drug discovery, neurotoxic agents, and neurodegeneration.

### REFERENCES

Alabed YZ, Grados-Munro E, Ferraro GB, Hsieh SH, Fournier AE (2006) Neuronal responses to myelin are mediated by rho kinase. J Neurochem 96:1616-1625.
Baraban SC, Bellingham MC, Berger AJ, Schwartzkroin PA (1997) Osmolarity modulates Pottasium channel function on rat hippocampal interneurons but not CA1 pyramidal neurons. Journal of Physiology 498:679-689.
Barone FC, White RF, Spera PA, Ellison J, Currie RW, Wang X, Feuerstein GZ, Rothwell NJ (1998) Ischemic Preconditioning and Brain Tolerance: Temporal Histological and Functional Outcomes, Protein Synthesis Requirement, and Interleukin-1 Receptor Antagonist and Early Gene Expression • Editorial Comment. Stroke 29:1937-1951.
Bikfalvi A, Klein S, Pintucci G, Rifkin DB (1997) Biological Roles of Fibroblast Growth Factor-2. Endocr Rev 18:26-45.
Boldin SA, Futerman AH (2000) Up-regulation of Glucosylceramide Synthesis upon Stimulation of Axonal Growth by Basic Fibroblast Growth Factor. EVIDENCE FOR POST-TRANSLATIONAL MODIFICATION OF GLUCOSYLCERAMIDE SYNTHASE. J Biol Chem 275:9905-9909.
Bousse L (1996) Whole cell biosensors. Sens Actuators, B 34:270-275.
Brewer GJ (1997) Isolation and culture of adult rat hippocampal neurons. Journal of Neuroscience Methods 71:143-155.
Brewer GJ (1999) Regeneration and proliferation of embryonic and adult rat hippocampal neurons in culture. Exp Neurol 159:237-247.
Charpentier Aea (1993) RRR-alpha-tocopheryl succinate inhibits proliferation and enhances secretion of transforming growth factor-beta (TGF-beta) by human breast cancer cells. Nutrition and Cancer 19:225-239.
Cunningham JJ, Roussel MF (2001) Cyclin-dependent kinases inhibitors in the development of the central nervous system. Cell Growth Differentiation 12:387-396.
Cuppini R, Ciaroni S, Cecchini T, Ambrogini P, Ferri P, Del Grande P, Papa S (2001) Alpha-tocopherol controls cell proliferation in the adult rat dentate gyrus. Neurosci Lett 303:198-200.
Das M, Bhargava N, Gregory C, Riedel L, Molnar P, Hickman JJ (2005) Adult rat spinal cord culture on an organosilane surface in a novel serum-free medium. In Vitro Cell Dev Biol Anim 41:343-348.
Das M, Patil S, Bhargava N, Kang JF, Riedel LM, Seal S, Hickman JJ (2007) Auto-catalytic ceria nanoparticles offer neuroprotection to adult rat spinal cord neurons. Biomaterials 28:1918-1925.
Dhavan R, Tsai L (2001) A decade of CDK5. National Review of Molecular Cell Biology 2:749-759.
Di Giovanni S, Movsesyan V, Ahmed F, Cernak I, Schinelli S, Stoica B, Faden AI (2005) Cell cycle inhibition provides neuroprotection and reduces glial proliferation and scar formation after traumatic brain injury. PNAS 102:8333-8338.
Dimitrova DS, Gilbert DM (2000) Temporally coordinated assembly and disassembly of replication factories in the absence of DNA synthesis. Nature Cell Biology 2:686-694.
Edwards D, Das M, Molnar P, Hickman JJ (2010) Addition of glutamate to serum-free culture promotes recovery of electrical activity in adult hippocampal neurons in vitro. Journal of Neuroscience Methods 190:155-163.
Fishman RA, Silverberg GD (2002) The cerebrospinal fluid production rate is reduced in dementia of the Alzheimer's type. Neurology 58:1866-.
Ginsberg SD (2005) Glutamatergic Neurotransmission Expression Profiling in the Mouse Hippocampus After Perforant-Path Transection. Am J Geriatr Psychiatry 13:1052-1061.
Goodyear S, Sharma MC (2007) Roscovitine regulates invasive breast cancer cell (MDA-MB231) proliferation and survival through cell cycle regulatory protein cdk5. Exp Mol Pathol 82:25-32.
Goodyear SM, Sharma MC (2005) Roscovitine induced cell death is mediated through specific inhibition of cell cycle regulatory protein cdk5. AACR Meeting Abstracts 2005:1045-d-1046.
Harms H, Wells MC, van der Meer JR (2006) Whole-cell living biosensors--are they ready for environmental application? Appl Microbiol Biotechnol 70:273-280.
Heiduschka P, Thanos S (1998) Impantable bioelectronic interfaces for lost nerve functions. Prog Neurobiology 55.
Herrup K, Yang Y (2007) Cell cycle regulation in the postmitotic neuron: oxymoron or new biology? Nat Rev Neurosci 8:368-378.
Hickman JJ, Spargo BJ, Testoff MA, Nielsen TB, Stenger DA, Rudolf AS (1994) Spatially Controlled Adhesion, Spreading, and Differentiation of Endothelial Cells on Self-Assembled Molecular Monolayers. PNAS 91:11070-11074.
Hung SC, Cheng H, Pan CY, Tsai MJ, Kao LS, Ma HL (2002) In vitro differentiation of size-sieved stem cells into electrically active neural cells. Stem Cells 20:522-529.
Katsuki H, Itsukaichi Y, Matsuki N (2000) Distinct signaling pathways involved in multiple effects of basic fibroblast growth factor on cultured rat hippocampal neurons. Brain Res 885:240-250.
Li B-S, Sun M-K, Zhang L, Takahashi S, Ma W, Vinade L, Kulkarni AB, Brady RO, Pant HC (2001) From the Cover: Regulation of NMDA receptors by cyclin-dependent kinase-5. PNAS 98:12742-12747.
Love S (2003) Neuronal expression of cell cycle-related proteins after brain ischemia in man. Neuroscience Letters 353:29-32.
Meijer L, Raymond E (2003) Roscovitine and other purines as kinase inhibitors. From starfish oocytes to clinical trials. Acc Chem Res 36:417-425.
Menn B, Bach S, Blevins TL, Campbell M, Meijer L, Timsit S (2010) Delayed treatment with systemic (S)-roscovitine provides neuroprotection and inhibits in vivo CDK5 activity increase in animal stroke models. PLoS One 5:e12117.
Monaco EA (2004) Recent evidence regarding a role for Cdk5 dysregulation in Alzheimer's disease. Current Alzheimer's Res 1 1:33-38.
Monyer H, Burnashev N, Laurie D, Sakmann B, Seeburg P (1994) Developmental and regional expression in the rat brain and functional properties of four NMDA receptors. Neuron 12:529-540.
Mulkey D, Henderson R, Putnam R, Dean J (2003) Hyperbaric oxygen and chemical oxidants stimulate CO2/H+ sensitive neurons in rat brain stem slices. Journal of Applied Physiology 95:910-921.
Mullen RJ, Buck CR, Smith AM (1992) NeuN, a neuronal specific nuclear protein in vertebrates. Development 116:201-211.
Muller FJ, Snyder EY, Loring JF (2006) Gene therapy: can neural stem cells deliver. Nature Reviews, Neuroscience 7:75.
Munaron L (2002) Calcium signalling and control of cell proliferation by tyrosine kinase receptors (review). Int J Mol Med 10:671-676.
Nagy Z, Esiri M, Cato A, Smith A (1997) Cell cycle marker in the hippocampus in Alzheimer's disease. Acta Neuropathol 94:6-15.
Oumata N, Bettayeb K, Ferandin Y, Demange L, Lopez-Giral A, Goddard ML, Myrianthopoulos V, Mikros E, Flajolet M, Greengard P, Meijer L, Galons H (2008) Roscovitine-derived, dual-specificity inhibitors of cyclin-dependent kinases and casein kinases 1. J Med Chem 51:5229-5242.
Padmanabhan J, Park DS, Greene LA, Shelanski ML (1999) Role of Cell Cycle Regulatory Proteins in Cerebellar Granule Neuron Apoptosis. J Neurosci 19:8747-8756.
Raley-Susman KM, Cragoe EJ, Jr., Sapolsky RM, Kopito RR (1991) Regulation of intracellular pH in cultured hippocampal neurons by an amiloride-insensitive Na+/H+ exchanger. J Biol Chem 266:2739-2745.
Ravenscroft M, Bateman K, Shaffer K, Schessler H, Jung DR, Schneider TW, Montgomery CB, Custer TL, Schaffner AE, Liu QY, Li YX, Barker JL, Hickman JJ (1998) Developmental neurobiology implications from fabrication and analysis of hippocampal neuronal networks on patterned silane- modified surfaces. Journal of American Chemical Society 120:12169-12177.
Rodan SB, Wesolowski G, Thomas KA, Yoon K, Rodan GA (1989) Effects of acidic and basic fibroblast growth factors on osteoblastic cells. Connect Tissue Res 20:283-288.
Schaffner AE, Barker JL, Stenger DA, Hickman JJ (1995) Investigation of the factors necessary for growth of hippocampal neurons in a defined system. Journal of Neuroscience Methods 62:111-119.
Scholzen T, Gerdes J (2000) The Ki-67 Protein: From the Known and the Unknown. Journal of Cellular Physiology 182:311-322.
Simpson ML, Sayler GS, Fleming JT, Applegate B (2001) Whole-cell biocomputing. Trends Biotechnol 19:317-323.
Stenger DA, Pike CJ, Hickman JJ, Cotman CW (1993) Surface determinants of neuronal survival and growth on self-assembled monolayers in culture. Brain Research 630:136-147.
Stenger DA, Hickman JJ, Bateman KE, Ravenscroft MS, Ma W, Pancrazio JJ, Shaffer K, Schaffner AE, Cribbs DH, Cotman CW (1998) Microlithographic determination of axonal/dendritic polarity in cultured hippocampal neurons. Journal of Neuroscience Methods 82:167-173.
Wesierska-Gadek J, Gueorguieva M, Horky M (2003) Dual action of cyclin-dependent kinase inhibitors: induction of cell cycle arrest and apoptosis. A comparison of the effects exerted by roscovitine and cisplatin. Pol J Pharmacol 55:895-902.
Yan Z, Chi P, Bibb JA, Ryan TA, Greengard P (2002) Roscovitine: a novel regulator of P/Q-type calcium channels and transmitter release in central neurons. J Physiol 540:761-770.
Yang Y, Mufson EJ, Herrup K (2003) Neuronal cell death is preceded by cell cycle events at all stages of Alzheimer's disease. Journal of Neuroscience 23:2557-2563.

## Claims

1. A method of producing a population of electrically active neurons, comprising
a) contacting adult neural cells with a growth factor under conditions to achieve confluence on a substrate;
b) contacting the adult neural cells of step a) with a cdk5 inhibitor; and
c) contacting the adult neural cells of step b) with a neurotransmitter,
wherein the adult neural cells develop into a population of electrically active neurons.

2. The method of Claim 1, wherein the adult neural cells are from normal adult neural tissue, from adult neural tissue having a known disease, or from adult neural tissue suspected of having a disease.

3. The method of Claim 2, wherein the adult neural tissue is brain tissue.

4. The method of Claim 1, wherein the substrate has been treated with N-1 [3-(trimethoxysilyl) propyl] -diethylenetriamine (DETA).

5. The method of Claim 1, wherein the growth factor comprises basic fibroblast growth factor (bFGF).

6. The method of Claim 1, further comprising maintaining electrically active neurons in a maintenance medium comprising a cdk5 factor inhibitor, Neurobasal-A, B27, Glutamax, an antibiotic/antimycotic, brain-derived neurotrophic factor, NT-3, basic fibroblast growth factor, and insulin-like growth factor.

7. The method of Claim 1, wherein the cdk5 inhibitor is roscovitine, butryolactone, or indolinone A.

8. The method of Claim 1 wherein the neurotransmitter is glutamate, acetylcholine, dopamine, serotonin, norepinephrine, epinephrine, GABA, prolactin, nitric oxide, histamine, vasopressin, oxytocin, an endocannabinoid, an endogenous opioid, glycine, or GHB.

9. The method of Claim 1, wherein the electrically active neurons express the neuronal markers MAP2 and NeuN.

## Patentansprüche

1. Verfahren für die Herstellung einer Population elektrisch aktiver Neurone, umfassend
a) In-Kontaktbringen von adulten neuralen Zellen mit einem Wachstumsfaktor unter Bedingungen, um Konfluenz auf einem Substrat zu erreichen;
b) In-Kontaktbringen der adulten neuralen Zellen von Schritt a) mit einem cdk5-Inhibitor; und
c) In-Kontaktbringen der adulten neuralen Zellen von Schritt b) mit einem Neurotransmitter, wobei die adulten neuralen Zellen sich in eine Population elektrisch aktiver Neurone entwickeln.

2. Verfahren nach Anspruch 1, wobei die adulten neuralen Zellen von normalem adulten neuralen Gewebe, von adultem neutralen Gewebe mit einer bekannten Krankheit oder von adultem neuralen Gewebe, von dem angenommen wird, dass es eine Krankheit aufweist, stammen.

3. Verfahren nach Anspruch 2, wobei das adulte neurale Gewebe Hirngewebe ist.

4. Verfahren nach Anspruch 1, wobei das Substrat mit N-1 [3-Trimethoxysilyl) propyl] -diethylentriamin (DETA) behandelt worden ist.

5. Verfahren nach Anspruch 1, wobei der Wachstumsfaktor basischen Fibroblasten-Wachstumsfaktor (bFGF) umfasst.

6. Verfahren nach Anspruch 1, des Weiteren umfassend Beibehalten elektrisch aktiver Neurone in einem Erhaltungsmedium, das einen cdk5-Faktor-Inhibitor, Neurobasal-A, B27, Glutamax, ein Antibiotikum/Antimykotikum, Hirn-abgeleiteten neurotrophischen Faktor, NT-3, basischen Fibroblasten-Wachstumsfaktor und Insulinartigen Wachstumsfaktor, umfasst.

7. Verfahren nach Anspruch 1, wobei der cdk5-Inhibitor Roscovitin, Butryolacton oder Indolinon A ist.

8. Verfahren nach Anspruch 1, wobei der Neurotransmitter Glutamat, Acetylcholin, Dopamin, Serotonin, Norepinephrin, Epinephrin, GABA, Prolactin, Stickstoffmonoxid, Histamin, Vasopressin, Oxytocin, ein Endocannabinoid, ein endogenes Opioid, Glycin oder GHB ist.

9. Verfahren nach Anspruch 1, wobei die elektrisch aktiven Neurone die neuronalen Marker MAP2 und NeuN exprimieren.

## Revendications

1. Procédé de production d'une population de neurones électriquement actifs, comprenant
a) la mise en contact de cellules neuronales adultes avec un facteur de croissance dans des conditions permettant d'obtenir une confluence sur un substrat ;
b) la mise en contact des cellules neuronales adultes de l'étape a) avec un inhibiteur de cdk5 ; et
c) la mise en contact des cellules neuronales adultes de l'étape b) avec un neurotransmetteur,
dans lequel les cellules neuronales adultes se développent en une population de neurones électriquement actifs.

2. Procédé selon la revendication 1, dans lequel les cellules neuronales adultes sont issues d'un tissu neuronal adulte normal, d'un tissu neuronal adulte présentant une maladie connue, ou d'un tissu neuronal adulte soupçonné de présenter une maladie.

3. Procédé selon la revendication 2, dans lequel le tissu neuronal adulte est un tissu cérébral.

4. Procédé selon la revendication 1, dans lequel le substrat a été traité avec de la N-1-[3-(triméthoxysilyl)propyl]-diéthylènetriamine (DETA).

5. Procédé selon la revendication 1, dans lequel le facteur de croissance comprend un facteur de croissance basique des fibroblastes (bFGF).

6. Procédé selon la revendication 1, comprenant en outre le maintien de neurones électriquement actifs dans un milieu de maintien comprenant un inhibiteur de facteur cdk5, du Neurobasal-A, du B27, du Glutamax, un antibiotique/antimycosique, un facteur neurotrophique dérivé du cerveau, du NT-3, un facteur de croissance basique des fibroblastes et un facteur de croissance analogue à l'insuline.

7. Procédé selon la revendication 1, dans lequel l'inhibiteur de cdk5 est la roscovitine, la butryolactone ou l'indolinone A.

8. Procédé selon la revendication 1, dans lequel le neurotransmetteur est le glutamate, l'acétylcholine, la dopamine, la sérotonine, la norépinéphrine, l'épinéphrine, le GABA, la prolactine, l'oxyde nitrique, l'histamine, la vasopressine, l'ocytocine, un endocannabinoïde, un opioïde endogène, la glycine ou le GHB.

9. Procédé selon la revendication 1, dans lequel les neurones électriquement actifs expriment les marqueurs neuronaux MAP2 et NeuN.
